# EUROPEAN PATENT APPLICATION

(11) **EP 1 621 555 A1**
(43) Date of publication of application: **01.02.2006**
(21) Application number: 04728051.6
(22) Date of filing: 16.04.2004
(51) Int. Cl.: C07K 19/00, C07K 16/00, C12N 15/62

(54) **IMMUNOGEN, COMPOSITION FOR IMMUNOLOGICAL USE, AND METHOD OF PRODUCING ANTIBODY USING THE SAME**

(30) Priority: 18.04.2003 JP 2003114503
(71) Applicant: Chiba, Joe, Yokohama-shi, Kanagawa 2470006 (JP)
(72) Inventor: IZUMOTO, Yoshitaka, Mishima-gun, Osaka 6188589 (JP); HATA, Jun-ichi, Mishima-gun, Osaka 6188589 (JP); IDENO, Akira, Mishima-gun, Osaka 6188589 (JP); FURUTANI, Masahiro, Mishima-gun, Osaka 6188589 (JP)
(74) Representative: Grund, Martin
(86) International application number: PCT/JP2004/005501
(87) International publication number: WO 2004/092221

(57) **Abstract**

It is intended to provide an immunogen and a composition for immunological use by which a sufficient immune response is induced even by an antigen protein which has been considered unavailable to induce any effective immune responses for various reasons, and a method of producing an antibody using the same. The immunogen containing a fusion protein including the full-length or a part of a desired antigen protein and a folding factor or its subunit linked thereto via at least one peptide bond. Owing to the function of the folding factor, the antigen protein contained in the fusion protein is obtained as a soluble protein having a correct stereostructure. It is also possible to protect the antigen protein and from rapid decomposition in the blood of an animal having been inoculated therewith. The immunogen induces a higher immune response for an animal than an antigen protein alone. Examples of the folding factor include chaperonins and PPIases.

SEQUENCE LISTING

<110>  SEKISUI CHEMICAL CO., LTD.

<120>  IMMUNOGEN, COMPOSITION FOR IMMUNOLOGICAL USE, AND METHOD OF PRODUCING
ANTIBODY USING THE SAME

<130>  471-2

<150>  JP2003-114503
<151>  2003-04-18

<160>  20

<170>  PatentIn version 3.1

<210>  1
<211>  1647
<212>  DNA
<213>  Escherichia coli

<400>  1
atggcagcta aagacgtaaa attcggtaac gacgctcgtg tgaaaatgct gcgcggcgta      60

aacgtactgg cagatgcagt gaaagttacc ctcggtccaa aaggccgtaa cgtagttctg     120

gataaatctt tcggtgcacc gaccatcacc aaagatggtg tttccgttgc tcgtgaaatc     180

gaactggaag acaagttcga aaatatgggt gcgcagatgg tgaaagaagt tgcctctaaa     240

gcaaacgacg ctgcaggcga cggtaccacc actgcaaccg tactggctca ggctatcatc     300

actgaaggtc tgaaagctgt tgctgcgggc atgaacccga tggacctgaa acgtggtatc     360

gacaaagcgg ttaccgctgc agttgaagaa ctgaaagcgc tgtccgtacc atgctctgac     420

tctaaagcga ttgctcaggt tggtaccatc tccgctaact ccgacgaaac cgtaggtaaa     480

ctgatcgctg aagcgatgga caaagtcggt aaagaaggcg ttatcaccgt tgaagacggt     540

accggtctgc aggacgaact ggacgtggtt gaaggtatgc agttcgaccg tggctacctg     600

tctccttact tcatcaacaa gccggaaact ggcgcagtag aactggaaag cccgttcatc     660

ctgctggctg acaagaaaat ctccaacatc cgcgaaatgc tgccggttct ggaagctgtt     720

gccaaagcag gcaaaccgct gctgatcatc gctgaagatg tagaaggcga agcgctggca     780

actgctgttg ttaacaccat tcgtggcatc gtgaaagtcg ctgcggttaa agcaccgggc     840

ttcggcgatc gtcgtaaagc tatgctgcag gatatcgcaa ccctgactgg cggtaccgtg     900

atctctgaag agatcggtat ggagctggaa aaagcaaccc tggaagacct gggtcaggct     960

aaacgtgttg tgatcaacaa agacaccacc actatcatcg atggcgtggg tgaagaagct    1020

gcaatccagg gccgtgttgc tcagatccgt cagcagattg aagaagcaac ttctgactac    1080

gaccgtgaaa aactgcagga acgcgtagcg aaactggcag gcggcgttgc agttatcaaa    1140

gtgggtgctg ctaccgaagt tgaaatgaaa gagaaaaaag cacgcgttga agatgccctg    1200

cacgcgaccc gtgctgcggt agaagaaggc gtggttgctg gtggtggtgt tgcgctgatc    1260

cgcgtagcgt ctaaactggc tgacctgcgt ggtcagaacg aagaccagaa cgtgggtatc    1320

aaagttgcac tgcgtgcaat ggaagctccg ctgcgtcaga tcgtattgaa ctgcggcgaa    1380

gaaccgtctg ttgttgctaa caccgttaaa ggcggcgacg gcaactacgg ttacaacgca 1440

gcaaccgaag aatacggcaa catgatcgac atgggtatcc tggatccaac caaagtaact 1500

cgttctgctc tgcagtacgc agcttctgtg gctggcctga tgatcaccac cgaatgcatg 1560

gttaccgacc tgccgaaaaa cgatgcagct gacttaggcg ctgctggcgg tatgggcggc 1620

atgggtggca tgggcggcat gatgtaa 1647

<210> 2
<211> 42
<212> DNA
<213> Artificial

<220>
<223> Designed oligonucleotide primer for PCR

<400> 2
agccactagt gcagctaaag acgtaaaatt cggtaacgac gc 42

<210> 3
<211> 44
<212> DNA
<213> Artificial

<220>
<223> Designed oligonucleotide primer for PCR

<400> 3
agcctctaga catcatgccg ccgatgccac ccatgccgcc cata 44

<210> 4
<211> 40
<212> DNA
<213> Artificial

<220>
<223> Designed oligonucleotide linker

<400> 4
ggtctagatg gaagttctgt tccagggtcc ggagatctcc 40

<210> 5
<211> 40
<212> DNA
<213> Artificial

<220>
<223> Designed oligonucleotide linker

<400> 5
ggagatctcc ggaccctgga acagaacttc catctagacc 40

<210> 6
<211> 1266
<212> DNA
<213> Homo sapiens

<400> 6
atggatgtgc tcagccctgg tcagggcaac aacaccacat caccaccggc tccctttgag 60

accggcggca acactactgg tatctccgac gtgaccgtca gctaccaagt gatcacctct 120

ctgctgctgg gcacgctcat cttctgcgcg gtgctgggca atgcgtgcgt ggtggctgcc 180
atcgccttgg agcgctccct gcagaacgtg gccaattatc ttattggctc tttggcggtc 240
accgacctca tggtgtcggt gttggtgctg cccatggccg cgctgtatca ggtgctcaac 300
aagtggacac tgggccaggt aacctgcgac ctgttcatcg ccctcgacgt gctgtgctgc 360
acctcatcca tcttgcacct gtgcgccatc gcgctggaca ggtactgggc catcacggac 420
cccatcgact acgtgaacaa gaggacgccc cggccgcgtg cgctcatctc gctcacttgg 480
cttattggct tcctcatctc tatcccgccc atcctgggct ggcgcacccc ggaagaccgc 540
tcggaccccg acgcatgcac cattagcaag gatcatggct acactatcta ttccaccttt 600
ggagctttct acatcccgct gctgctcatg ctggttctct atgggcgcat attccgagct 660
gcgcgcttcc gcatccgcaa gacggtcaaa aaggtggaga agaccggagc ggacacccgc 720
catggagcat ctcccgcccc gcagcccaag aagagtgtga atggagagtc ggggagcagg 780
aactggaggc tgggcgtgga gagcaaggct gggggtgctc tgtgcgccaa tggcgcggtg 840
aggcaaggtg acgatggcgc cgccctggag gtgatcgagg tgcaccgagt gggcaactcc 900
aaagagcact tgcctctgcc cagcgaggct ggtcctaccc cttgtgcccc cgcctctttc 960
gagaggaaaa atgagcgcaa cgccgaggcg aagcgcaaga tggccctggc ccgagagagg 1020
aagacagtga agacgctggg catcatcatg ggcaccttca tcctctgctg gctgcccttc 1080
ttcatcgtgg ctcttgttct gcccttctgc gagagcagct gccacatgcc caccctgttg 1140
ggcgccataa tcaattggct gggctactcc aactctctgc ttaaccccgt catttacgca 1200
tacttcaaca aggactttca aaacgcgttt aagaagatca ttaagtgtaa cttctgccgc 1260
cagtga 1266

<210> 7
<211> 29
<212> DNA
<213> Artificial

<220>
<223> Designed oligonucleotide primer for PCR

<400> 7
agccagatct atggatgtgc tcagccctg 29

<210> 8
<211> 29
<212> DNA
<213> Artificial

<220>
<223> Designed oligonucleotide primer for PCR

<400> 8
agccctcgag ctggcggcag aagttacac 29

<210> 9
<211> 1641

<212>  DNA
<213>  Thermococcus sp. KS-1

<400>  9
atggcccagc ttgcaggcca gccagttgtt attctacctg agggaactca gaggtacgtt      60

ggaagggacg cccagaggct caacattctt gctgccagga ttatagccga gacggttaga     120

accacccttg gaccaaaggg aatggacaag atgctcgttg acagcctcgg cgacatcgtc     180

atcaccaacg acggtgcaac cattctcgac gagatggaca tccagcaccc tgctgctaag     240

atgatggttg aggttgctaa gactcaggat aaggaggctg gtgatggtac tactactgcg     300

gttgttattg ctggtgagct tctgaggaag gctgaggagc ttctcgacca gaacattcac     360

ccgagcataa tcatcaaggg ctacgccctc gcagcagaga aagcccagga aatactcgac     420

gagatagcca aggacgttga cgtcgaggac agggagattc tcaagaaggc cgcggtcacc     480

tccatcaccg gaaaggccgc cgaggaggag agggagtacc tcgctgagat agcagttgag     540

gccgtcaagc aggttgccga gaaggttggc gagacctaca aggtcgacct cgacaacatc     600

aagttcgaga agaaggaagg tggaagcgtc aaggacaccc agctcataaa gggtgtcgtc     660

atcgacaagg aggtcgtcca cccaggcatg ccgaagaggg tcgagggtgc taagatcgcc     720

ctcatcaacg aggcccttga ggtcaaggag actgagaccg acgccgagat caggatcacc     780

agcccggagc agctccaggc cttccttgag caggaggaga agatgctcag ggagatggtc     840

gacaagatca aggaggtcgg cgcgaacgtc gtgttcgtcc agaagggcat tgacgacctt     900

gcccagcact acctggccaa gtacggcata atggcagtca ggagggtcaa gaagagcgac     960

atggagaagc tcgccaaggc cactggagct aagatcgtca ccaacgtccg cgacctcacc    1020

ccggaggacc tcggtgaggc cgagctcgtc gagcagagga aggtcgccgg cgagaacatg    1080

atcttcgtcg agggctgcaa gaacccgaag gcagtgacaa tactcatcag gggcggtacc    1140

gagcacgtcg ttgacgaggt cgagagggcc ctcgaggatg ccgtcaaggt cgtcaaggac    1200

atcgtcgagg acggcaagat cgtcgccgcc ggcggtgctc cggagatcga gctcagcatc    1260

aggctcgacg agtacgcgaa ggaggtcggc ggcaaggagc agctcgccat cgaggccttt    1320

gcagaggccc tcaaggtcat tccgaggacc ctcgccgaga acgccggtct cgacccgatc    1380

gagaccctcg ttaaggtcat cgccgcccac aaggagaagg gaccgaccat cggtgttgac    1440

gtcttcgagg gcgagccggc cgacatgctc gagcgcggcg tcatcgcccc ggtcagggtt    1500

ccgaagcagg ccatcaagag cgccagcgag gccgccataa tgatcctcag gatcgacgac    1560

gtcatcgccg ccagcaagct cgagaaggac aaggagggcg gcaagggcgg tagcgaggac    1620

ttcggaagcg atctcgactg a                                              1641

<210>  10
<211>  35
<212>  DNA
<213>  Artificial

<220>
<223>  Designed oligonucleotide primer for PCR

<400>  10
ggagatctgc ccagcttgca ggccagccag ttgtt                                35

<210>  11
<211>  35
<212>  DNA
<213>  Artificial

<220>
<223>  Designed oligonucleotide primer for PCR

<400>  11
ccggatccga cgagatcgct tccgaagtcc tcgct                                35

<210>  12
<211>  76
<212>  DNA
<213>  Artificial

<220>
<223>  Designed oligonucletide linker

<400>  12
ggcatatggg atccctggtt ccgcgtggct caagatctct cgagcatcgc catcgccatc     60

gctaatagga attccc                                                     76

<210>  13
<211>  76
<212>  DNA
<213>  Artificial

<220>
<223>  Designed oligonucleotide linker

<400>  13
gggaattcct attagcgatg gcgatggcga tgctcgagag atcttgagcc acgcggaacc     60

agggatccca tatgcc                                                     76

<210>  14
<211>  480
<212>  DNA
<213>  Thermococcus sp. KS-1

<400>  14
atgaaagttg aagctggtga ttatgttctc ttccactacg ttggaaggtt cgaggatgga     60

gaagtttttg acacaagcta cgaggagata gccagagaga atggcattct cgtcgaggag    120

agggagtacg gcccaatgtg ggtcaggata ggcgtcggtg agatcatccc tggcctcgat    180

gaagccataa ttggcatgga agctggagag aagaagaccg tgaccgttcc ccccgagaag    240

gcttacggaa tgccgaaccc agagcttgta atctccgttc caagggaaga attcacaaag    300

gccggccttg aaccccagga aggtctctac gtcatgaccg attctggcat agccaagata    360

gtttccgttg gagagagcga ggtatccctt gacttcaacc acccgctagc aggtaagacc    420

ctagtctttg aggtagaagt catagaagta aaaaaggccg aagaggactc agaagcttag    480

<210> 15
<211> 28
<212> DNA
<213> Artificial

<220>
<223> Designed oligonucleotide primer for PCR

<400> 15
ggccatggga aaagttgaag ctggtgat 28


<210> 16
<211> 26
<212> DNA
<213> Artificial

<220>
<223> Designed oligonucleotide primer for PCR

<400> 16
ccactagtag cttctgagtc ctcttc 26


<210> 17
<211> 40
<212> DNA
<213> Artificial

<220>
<223> Designed oligonucleotide linker

<400> 17
ggactagtct ggttccgcgt ggatcccata tggaatccgg 40


<210> 18
<211> 40
<212> DNA
<213> Artificial

<220>
<223> Designed olligonucleotide linker

<400> 18
ccggattcca tatgggatcc acgcggaacc agactagtcc 40


<210> 19
<211> 24
<212> DNA
<213> Artificial

<220>
<223> Designed oligonucleotide primer for PCR

<400> 19
ggcatatgga tgtgctcagc cctg 24


<210> 20
<211> 45
<212> DNA
<213> Artificial

<220>
<223> Designed oligonucleotide primer for PCR

<400> 20
ccgagctcgc gatggcgatg gcgatgctgg cggcagaagt tacac 45

## Description

### TECHNICAL FIELD

The present invention relates to an immunogen, a composition for immunological use, and a method of producing an antibody using the same, more particularly, to an immunogen including a fusion protein composed of a desired antigen protein and a folding factor, a composition containing the fusion protein for immunological use, and a method of producing an antibody using the same. According to the present invention, a sufficient immune response is induced even by an antigen protein which has been considered unavailable to induce any effective immune responses for various reasons.

### BACKGROUND ART

With the progress of biotechnology, the importance of antibodies is more and more increasing. For example, various types of immunoassay techniques such as enzyme immunoassay and radioimmunoassay and further affinity chromatography techniques useful for protein purification utilize an antigen-antibody reaction, a specific reaction of antibodies. An antibody is prepared by inoculation of an animal with an antigen as an immunogen, and inducing an immune response in the animal which results in production of the antibody. Recently, a transformant having a gene encoding an antigen protein wherein the gene is isolated by recombinant DNA techniques is cultured, thereby obtaining the antigen protein, not by preparation and purification of an antigen protein from a sample derived from a living body.

However, even though an antigen protein obtained in this way is used as an immunogen, desired antibodies may not be obtained in some cases. The examples are as follows:
(a) When the antigen protein is toxic to the host cells, transformants to produce the antigen protein may be killed, resulting in difficulty of preparation of enough amount of the antigen protein.
(b) When the antigen protein does not have a correct stereostructure in the host cell, the antigen protein may be obtained only as an abnormal protein.
(c) When the antigen protein is an insoluble protein containing plenty of hydrophobic amino acids, the antigen protein as an immunogen may not induce a sufficient immune response.
(d) When the antigen protein as an immunogen is rapidly decomposed in the blood of an inoculated animal, the antigen protein may not induce a sufficient immune response.

To solve these problems, exploitation of a peptide antigen that is an artificially synthesized peptide corresponding to a region to be expected as an epitope or addition of lipids to an antigen protein to induce a sufficient immune response has been attempted. However, methods to solve all above-described problems have not been developed (for example, see WO02/052029). Especially, no attempt to solve the problem that the antigen protein as an immunogen is rapidly decomposed in the blood of an inoculated animal has been made.

An object of the present invention is to provide an immunogen and a composition for immunological use whereby a sufficient immune response is induced even by an antigen protein which has been considered unavailable to induce any effective immune responses for various reasons, and a method of producing an antibody using the same.

### DICLOSURE OF THE INVENTION

As the result of diligent researches, the inventors have found that the use of a fusion protein composed of an antigen protein and a folding factor as an immunogen provides the following effects:
(a) Even though the antigen protein is toxic to the host cells, transformants to produce the antigen protein are protected from being killed. Therefore, it is easy to prepare enough amount of the antigen protein contained in the fusion protein as an immunogen.
(b) Because the antigen protein tends to have a correct stereostructure easily in the fusion protein, an immune response is induced more correctly.
(c) Even though the antigen protein contains plenty of hydrophobic amino acids and tends to be insoluble in itself, the antigen protein in the fusion protein is soluble. Therefore, the fusion protein as an immunogen induces a sufficient immune response against the antigen protein.
(d) The fusion protein is not rapidly decomposed in the blood of an inoculated animal, thereby inducing a sufficient immune response against the antigen protein.
(e) The fusion protein induces an immune response against the antigen protein in an animal more efficiently than the antigen protein alone.

The summaries of the present invention are as follows:
(1) An immunogen for inducing an immune response to a desired antigen protein, the immunogen including a fusion protein composed of one selected from the full-length and a part of the antigen protein and one selected from a folding factor and its subunit linked thereto via at least one peptide bond;
(2) The immunogen as described in (1) wherein the folding factor is a chaperonin consisting of a plurality of chaperonin subunits;
(3) The immunogen as described in (2) wherein at least two of the chaperonin subunits are serially linked to one another via peptide bonds;
(4) The immunogen as described in (2) or (3) wherein the antigen protein is linked to the N-terminus and/or the C-terminus of the chaperonin subunit;
(5) The immunogen as described in (3) or (4) wherein the antigen protein is linked between the chaperonin subunits;
(6) The immunogen as described in one of (2)-(5) being provided with an amino acid sequence to be cleaved by a protease between the chaperonin subunit and the antigen protein;
(7) The immunogen as described in one of (3)-(6) being provided with an amino acid sequence to be cleaved by a protease between the chaperonin subunits;
(8) The immunogen as described in one of (2)-(7) wherein the chaperonin subunit is derived from one selected from a group consisting of bacteria, archaea and eukaryotes;
(9) The immunogen as described in one of (2)-(8) wherein the antigen protein is accommodated in a chaperonin ring formed by the chaperonin subunits;
(10) The immunogen as described in (9) wherein the chaperonin ring is consisting of 5 to 10 chaperonin subunits;
(11) The immunogen as described in (9) or (10) having two chaperonin rings non-covalently associated on each other's ring plane or each other's side;
(12) The immunogen as described in (1) wherein the folding factor is a foldase;
(13) The immunogen as described in (12) wherein the antigen protein is linked to the N-terminus and/or the C-terminus of the foldase;
(14) The immunogen as described in (12) or (13) wherein the foldase is a PPIase;
(15) The immunogen as described in (14) wherein the PPIase is derived from one selected from a group consisting of Escherichia coli and archaea;
(16) The immunogen as described in one of (1)-(15) wherein the antigen protein is serotonin receptor 5-HT1aR;
(17) The immunogen as described in (16) wherein the fusion protein includes either the full-length of serotonin receptor 5-HT1aR or a partial protein consisting of 6 or more amino acid residues thereof;
(18) The immunogen as described in one of (1)-(17) being produced by transcription and translation of a fusion gene including a gene encoding one selected from the full-length and a part of the antigen protein and a gene encoding one selected from the folding factor and its subunit;
(19) The immunogen as described in (18) wherein the gene encoding a part of the antigen protein is a gene encoding a partial protein consisting of 6 or more amino acid residues of the antigen protein;
(20) A composition for immunological use being prepared by mixing of the immunogen as described in one of (1)-(19) with an adjuvant;
(21) A method of producing an antibody, the method including the steps of immunizing an animal except human with the immunogen as described in one of (1)-(19) and obtaining an antibody specific to the antigen protein from the animal; and
(22) A method of producing an antibody, the method including the steps of immunizing an animal except human with the composition as described in (20) and obtaining an antibody specific to the antigen protein from the animal.

### BRIEF DESCRIPTION OF THE DRAWINGS

Figs. 1A to E are illustrations showing first examples of combinations of antigen proteins and chaperonin subunits in a chaperonin-antigen protein complex;
Figs. 2A to D are illustrations showing second examples of combinations of antigen proteins and chaperonin subunits in a chaperonin-antigen protein complex;
Figs. 3A to D are illustrations showing third examples of combinations of antigen proteins and chaperonin subunits in a chaperonin-antigen protein complex;
Figs. 4A to D are illustrations showing fourth examples of combinations of antigen proteins and chaperonin subunits in a chaperonin-antigen protein complex;
Figs. 5A to D are illustrations showing fifth examples of combinations of antigen proteins and chaperonin subunits in a chaperonin-antigen protein complex;.
Fig. 6 is an illustration showing a constitution of the vector pTrc(GroEL)7;
Fig. 7 is an illustration showing a constitution of the vector pTF(TCPβ)8; Fig. 8 is an illustration showing a constitution of the vector TcFKfusion2; and
Fig. 9 is a schematic illustration of a stereostructure of Escherichia coli chaperonin (GroEL).

### DESCRIPTION OF THE PREFERRED EMBODIMENTS

The immunogen of the present invention includes a fusion protein composed of the full-length or a part of an antigen protein and a folding factor or its subunit linked thereto via at least peptide bond.

Herein, "folding factor" denotes a series of proteins which assist polypeptide folding. Folding factors are generally classified into molecular chaperones and foldases.

Expressions of most of molecular chaperones are induced upon application of stress such as heat shock to cells. Molecular chaperones assist protein folding and contribute to structural stabilization of proteins in the presence or absence of ATP, an energy substance. Examples of the molecular chaperones include chaperonins, Hsp70 systems, small heat shock proteins, Hsp90, and prefoldins. In the immunogen of the present invention, any molecular chaperone is applicable as a folding factor. Each of the molecular chaperones applicable in the present invention will be described below.

First, chaperonins will be described. A chaperonin is a complex protein composed of a plurality of subunits (chaperonin subunits) with a molecular weight of about 60 kDa. The chaperonin occurs in every living thing such as bacteria, archaea and eukaryotes, and has a function of assisting protein folding and protecting proteins from denaturation.

Chaperonins are classified into group 1-type chaperonins and group 2-type chaperonins. The group 1-type chaperonins occur in bacteria or organelle of eukaryotes and require a co-factor called "co-chaperonin" that is a ring complex formed by proteins with a molecular weight of about 10 kDa. The group 2-type chaperonins occur in eukaryotic cytosol or archaea, but their structures and functions are not well known and proteins corresponding to the co-chaperonins of the group 1-type chaperonin have not been found yet (Gupta, Mol. Microbiol., 15, p1-, 1995). Both types of the chaperonins are applicable in the immunogen of the present invention. GroEL derived from Escherichia coli as the group 1-type chaperonin and TCP derived from archaea as the group 2-type chaperonin may be used, for example.

A typical chaperonin is a complex protein having two ring complexes (chaperonin rings) each consisting of 5 to 10 chaperonin subunits arranged annularly, that is, composed of 10 to 20 chaperonin subunits. The two chaperonin rings are non-covalently associated on each other's ring plane so as to form a two-layer chaperonin ring structure. The chaperonin accommodates a polypeptide or a denatured protein in its ring structure to promote protein folding reaction with consuming nucleotide triphosphate such as ATP. Another chaperonin having one chaperonin ring with a one-layer ring structure (single ring chaperonin) is also known. The single ring chaperonin not only occurs in some kinds of living things, but also is artificially made by means of a protein engineering technique such as amino acid substitution. One of the reasons why the immunogen of the present invention has the actions as described in (a)-(d) is due to that the antigen protein is accommodated in the chaperonin.

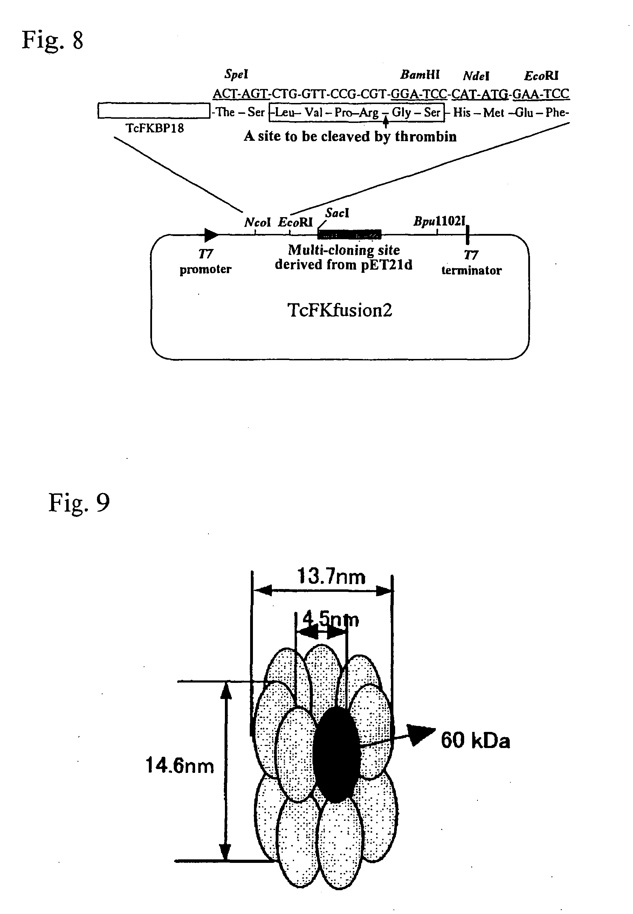

As shown in Fig. 9, GroEL, a chaperonin derived from Escherichia coli, has a cavity with an inner diameter of 4.5 nm and a height of 14.5 nm. The cavity of the chaperonin ring has enough space for accommodating a globular protein with a molecular weight of 60 kDa. The chaperonin has a function of transiently accommodating an polypeptide or a denatured protein in the cavity, thereby assisting the polypeptide or the protein to have a folded structure and stabilizing the structure.

Recently, stereostructures of chaperonins derived from various cells have been revealed in detail by X-ray crystallographic analysis. The structure of a chaperonin formed by interactions between the adjacent chaperonin subunits and between the chaperonin rings is maintained by non-covalent interactions such as a hydrophobic or ionic interaction among amino acids. The C-terminus and the N-terminus of the chaperonin subunit are located at the cavity side with high flexibilities. Especially, it is known that at least 23 amino acid residues of the C-terminus of GroEL, a chaperonin derived from Escherichia coli, have a high flexible structure.

When the chaperonin is present at a high concentration of not less than 1 mg/mL in the presence of Mg-ATP, two-layer chaperonin rings may further be non-covalently associated to one another on each other's ring plane or each other's side to assemble into a fibrous structure (Trent J. D. et al., Proc. Natl. Acad. Sci. U.S.A., 94, 5383-5388, 1997, and, Furutani M. et al., J. Biol. Chem., 273, 28399-28407, 1998).

In the immunogen of the present invention, it is preferable to use a chaperonin having two chaperonin rings, each composed of chaperonin subunits, non-covalently associated on each other's ring plane so as to form a two-layer ring structure, or a chaperonin having two chaperonin rings non-covalently associated on each other's ring plane or on each other's side so as to assemble into a fibrous structure. However, a single ring chaperonin may be also used.

Chaperonins derived from bacteria or archaea are produced in a host cell such as Escherichia coli by recombinant DNA techniques. When Escherichia coli is used as a host cell, for example, the chaperonins are easily produced in its soluble cytosol fraction in a large amount. Further, it has been confirmed that heterogeneous organism-derived chaperonins produced in Escherichia coli cells are self-assembled, thereby forming a complex with a two-layer ring structure, under electron microscope observation of the purified chaperonins produced in the Escherichia coli cytosol soluble fraction.

In the present invention, chaperonins may be derived from any organism such as bacteria, archaea and eukaryotes and may be native ones or artificial ones produced in an appropriate host cell by recombinant DNA techniques. Further, as long as the ability whereby a chaperonin is self-assembled into a ring structure is maintained, not only wild-type chaperonins but amino acid mutant chaperonins may be used. A mutant chaperonin with reduced associating ability of each chaperonin subunit may be also used to release an antigen protein accommodated in a chaperonin ring more rapidly, for example.

Molecular chaperones belonging to Hsp70 systems will be now described. A complex composed of a molecular chaperone belonging to Hsp70 systems and an antigen protein is suitable as an immunogen because the complex protects the antigen protein from irreversible aggregation.

Typical proteins belonging to Hsp70 systems include DnaK, DnaJ, and GrpE, all derived from Escherichia coli. Further, most of organisms, regardless of species, have homologues of DnaK, Dna J, and GrpE. Protein folding systems by DnaK/DnaJ/GrpE of Escherichia coli are well studied. A reaction mechanism described below is proposed. A nascent polypeptide biosynthesized in a ribosome binds to DnaK and further to DnaJ in the presence of ATP, so as to inhibit formation of irreversibly insoluble aggregates. Further, with dissociation of nucleotides depending on GrpE, the nascent polypeptide is also dissociated and then transferred to a folding system of chaperonins (Fink, Molecular chaperones in the life cycle of protein, MARCEL DEKKER, Inc., 1998). DnaK, DnaJ, GrpE, all derived from Escherichia coli, and their homologues having the same activity are applicable in the immunogen of the present invention.

Molecular chaperones belonging to small heat shock proteins will be now described. A small heat shock protein forms a simple complex, not a complicated and higher-order complex like a chaperonin having a ring structure. An antigen protein forms a complex with a small heat shock protein, and whereby the complex of the small heat shock protein surrounds the antigen protein to protect. Consequently, the complex induces an immune response more certainly without rapid decomposition of the antigen protein in the blood of an inoculated animal. It is reported that a small heat shock protein has a huge structure composed of about 24 to 32 subunits each with a molecular weight of about 15-30 kDa and has a molecular chaperone activity (Jakob, J. Biol. Chem., 268, 1517-, 1993). One of the small heat shock proteins, Hsp25, for example, is composed of 16 or more subunits associated with one another, (Ehrnsperger, M et al., The Journal of Biological Chemistry, vol. 274, No. 21, 14867-14874). A crystalline having high homology with the small heat shock proteins at its C-terminus region has also the same property as a small heat shock protein. A fusion protein composed of an antigen protein and a small heat shock protein is applicable as the immunogen of the present invention.

Molecular chaperones belonging to prefoldins will be now described. A prefoldin forms a simple complex as well as a small heat shock protein. An antigen protein forms a complex with a prefoldin, and whereby the complex of the prefoldin surrounds the antigen protein to protect. Consequently, the complex induces an immune response more certainly without rapid decomposition of the antigen protein in the blood of an inoculated animal. A prefoldin is a molecular chaperone found as a factor involving in protein folding of tubulin of eukaryotes. A prefoldin forms hexamer and has a chaperone activity interacting with denatured proteins in vitro (Sigert, Cell, 103, 621-, 2000). A fusion protein composed of an antigen protein and a prefoldin is applicable as the immunogen of the present invention.

Foldases, other folding factors, will be now described. A Foldase is a folding factor acting enzymatically. A fusion protein composed of a foldase and an antigen protein is suitable as an immunogen because the antigen protein in the fusion protein tend to have a correct stereostructure. Examples of foldases include peptidyl-prolyl cis-trans isomerases (hereinafter referred to as "PPIase") and protein disulfide isomerases. A PPIase is an enzyme catalyzing isomerization of prolyl peptide bonds in protein folding. A protein disulfide isomerase is an enzyme promoting formation of disulfide bonds. A foldase used in the immunogen of the present invention is not particularly limited, but PPIases are more preferably for use.

PPIases are classified into three types, an FK506 Binding Protein type (FKBP type), a cyclophilin type, a parvulin type based on sensitivity to its inhibitor. An FKBP-type PPIase is a PPIase, an activity of which is inhibited by FK506 that is one of immunosuppressive agents, and homologues thereof. A Cyclophilin-type PPIase is a PPIase having sensitivity to cyclosporine that is another immunosuppressive agent, or homologues thereof. On the other hand, a parvulin-type PPIase is a PPIase exhibiting no sensitivity to immunosuppressive agents, and an activity of which is inhibited by juglone. These three types of PPIases have little homology on their amino acid primary sequences. All the PPIases described above are applicable in the immunogen of the present invention.

Further, some kinds of PPIases have not only an original activity catalyzing isomerization of prolyl peptide bonds (PPIase activity) but also a molecular chaperone activity. Herein, "molecular chaperone activity" denotes to an activity of refolding a denatured protein to have an original natural structure, or an activity of inhibiting irreversible aggregations of a denatured protein. The molecular chaperone activity of the PPIases is different from the activity of molecular chaperones in that the activity of the PPIases dispense with nucleotide triphosphate like ATP for expression of the activity. PPIases having a molecular chaperone activity are more suitable for use in the immunogen of the present invention, because folding other proteins more correctly than PPIases with no molecular chaperone activity.

Examples of PPIases having a molecular chaperone activity include FKBP-type PPIases derived from archaea. Specifically, FKBP-type PPIases derived from archaea have both a PPIase activity and an ATP-independent molecular chaperone activity, thereby facilitating an antigen protein having a correct stereostructure more certainly by fusing the antigen protein with the FKBP-type PPIase. FKBP-type PPIases derived from archaea are classified into short-type ones with a molecular weight of about 16-18 kDa and long-type ones with a molecular weight of about 26-33 kDa. In the immunogen of the present invention, both types of PPIases are applicable, but short-type FKBP-type PPIases derived from archaea are suitable in the immunogen of the present invention because of having a higher molecular chaperone activity. Short-type FKBP-type PPIases derived from archaea have a low molecular weight, so as to have advantage to express the fusion protein with an antigen protein in a host cell by means of recombinant DNA techniques. The range of the above-described molecular weight is that of archaeal FKBP-type PPIases previously found, but archaeal FKBP-type PPIases used in the immunogen of the present invention are not limited to those having a molecular weight of the range, and any archaeal FKBP-type PPIases may be used as long as they belong to substantially the same group.

Archaea for obtaining FKBP-type PPIases used in the immunogen of the present invention are not particularly limited. Examples of archaea for obtaining short-type FKBP-type PPIases include thermophilic or hyperthermophilic archaea such as Methanococcus thermolithotrophicus, Thermococcus sp. KS-1, and Methanococcus jannaschii. Further, they include mesophilic archaea such as Methanosarcina mazei, Methanosarcina acetivorans ,and Methanosarcina barkeri (Maruyama, Front. Biosci. 5, 821-, 2000). On the other hand, examples of archaea for obtaining long-type FKBP-type PPIases include thermophilic or hyperthermophilic archaea such as Pyrococcus horikoshii, Aeropyrum pemix, Sulfolobus solfataricus, Methanococcus jannaschii, Archaeoglobus fulgidus, Methanobacterium autotrophicum, and Thermoplasma acidophilum. Further, they include mesophilic archaea such as Halobacterium cutirubrum (Maruyama, Front. Biosci. 5, 821-,2000).

Other types of PPIases having a molecular chaperone activity will be now described. FKBP-type PPIases include trigger factor-type ones, FkpA-type ones, and FKBP52-type ones. Cyclophilin-type PPIases include CyP40-type PPIases. Further, parvulin-type PPIases include Sur A-type PPlases.

A gene encoding a trigger factor-type PPIase has been found in genomes of almost all bacteria. Examples of the source organism for obtaining trigger factor-type PPIases include Escherichia coli, Mycoplasma genitalium, Bacillus subtilis, Salmonella enterica, Staphylococcus aureus, Mycobacterium leprae, Agrobacterium tumefacium, Lactococcus lactis, Campyrobacter jejuni, Streptococcus pyogenes, and Corynebacterium diphtheriae.

Both FkpA-type PPIases and SurA-type PPIases occur in periplasmic spaces of gram-negative bacteria such as Escherichia coli. Examples of the source organism for obtaining FkpA-type PPIases and SurA-type PPIases include Escherichia coli, Pyrobaculum aerophilium, Pseudomonas aeruginosa, Xylella fastidiosa, Neisseria meningitides, Mesorhizobium loti, Heamophilus influenzae, and Ralstonia solanacearum. FkpA-type PPIases and SurA-type PPIases derived from organisms other than those bacteria may be also used as long as the PPIases belong to the same group and have substantially the same activity. For example, a gene encoding a homologue thereof has been also found in genomes of eukaryotes such as yeast.

FKBP52-type PPIases and CyP40-type PPIases occur in eukaryotes. FKBP52-type PPIases, also called p59, HSP56 or the like, have a molecular weight of about 52 kDa. CyP40-type PPIases have a molecular weight of about 40 kDa. Examples of the source organism for obtaining FKBP52-type PPIases and CyP40-type PPIases include eukaryotes such as human, mouse, bovine, pig, rabbit, and rat. FKBP52-type PPIases and CyP40-type PPIases derived from organisms other than those eukalyotes may be also used as long as the PPIases belong to substantially the same group.

A partial polypeptide corresponding to a part of the above-described PPIases is also applicable as long as the polypeptide has the same activity as the full-length of the PPIases. Short-type FKBP-type PPIases derived from archaea, for example, have an FKBP domain involving in binding to FK506 and an IF (Insert in the flap) domain, the domains each managing a molecular chaperone activity. Therefore, a partial polypeptide containing these domains and having a PPIase activity is applicable as a PPIase having a molecular chaperone activity. On the other hand, long-type FKBP-type PPIases derived from archaea have a C-terminus domain managing a molecular chaperone activity in addition to an FKBP domain and an IF domain described above. Therefore, a partial polypeptide containing these domains and having a PPIase activity is applicable as a PPIase having a molecular chaperone activity. Introduction of an IF domain or a C-terminus domain into a PPIase with no molecular chaperone activity such as human FKBP52-type PPIases by protein engineering techniques prepares a chimeric PPIase having a molecular chaperone activity.

Fusion proteins contained in the immunogen of the present invention will be now described. As described above, the fusion protein is composed of the full-length or a part of an antigen protein and a folding factor or its subunit.

Firstly, the case of the folding factor employing a chaperonin will be described. The chaperonin is a complex composed of a plurality of chaperonin subunits, to one of which an antigen protein is linked via a peptide bond in the fusion protein used as the immunogen of the present invention. The antigen protein is linked to either site, the N-terminus or the C-terminus of the chaperonin subunit. When being linked to a chaperonin subunit linkage having chaperonin subunits linked to one another, the antigen protein is linked to one site selected from a group consisting of the N-terminus, the C-terminus, and a linking site of the chaperonin subunits. In the case of a plurality of the antigen proteins, the antigen proteins may be linked to a plurality of sites selected therefrom respectively. Especially, it is preferable that the sites to be linked are selected so that the antigen protein is certainly accommodated in the cavity of the chaperonin ring.

When an antigen protein has a quaternary structure consisting of a homodimer or a heterodimer, or higher oligomer in nature, it is preferable that one or two species of subunits constituting the antigen protein are respectively linked to the N-terminus and the C-terminus of chaperonin subunits linked to one another via peptide bonds. When a complex composed of an antigen protein and an archaeal chaperonin, wherein the antigen protein expresses its antigenicity by forming a dimer and wherein the archaeal chaperonin is composed of eight chaperonin subunits, is used as an immunogen, each subunit of the antigen protein may be linked respectively via peptide bonds to the N-terminus and the C-terminus of the chaperonin octamer having eight chaperonin subunits linked to one another (chaperonin subunit 8-times linkage). In this arrangement, each of the subunits is located in regions extremely close to one another in the chaperonin ring, thereby ensuring that the antigen protein easily has a quaternary structure composed of a dimer and express its stable antigenicity.

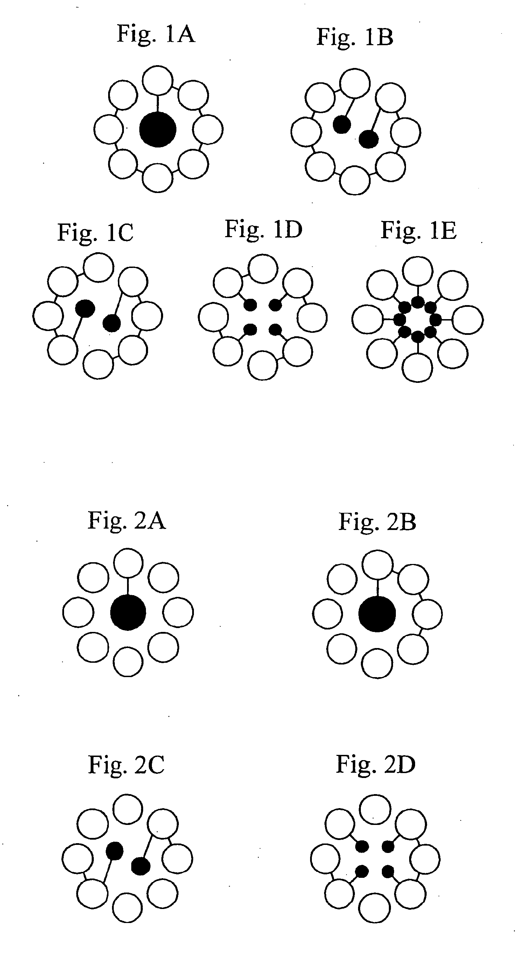

Examples of combinations of fusion proteins and chaperonin subunits in a complex composed of chaperonins and antigen proteins (hereinafter referred to as "chaperonin-antigen protein complex") will be described referring to Figs 1-5 taking a chaperonin of eight subunits as a model. In the figures, a white circle denotes a chaperonin subunit, a black circle denotes an antigen protein, and a straight line denotes a peptide bond. In the first examples, the chaperonin-antigen protein complex consists of only a fusion protein. Figures 1A to E show some schematic illustrations of the examples. The chaperonin-antigen protein complex as shown in Fig. 1A consists of a fusion protein composed of a chaperonin subunit 8-times linkage and an antigen protein linked thereto. The chaperonin-antigen protein complex as shown in Fig. 1B consists of a fusion protein composed of a chaperonin subunit 8-times linkage and two antigen proteins respectively linked to the N-terminus and the C-terminus thereof. The chaperonin-antigen protein complex as shown in Fig. 1C consists of two fusion proteins each composed of a chaperonin subunit 4-times linkage and an antigen protein linked thereto. The chaperonin-antigen protein complex as shown in Fig. 1D consists of four fusion proteins each composed of a chaperonin subunit 2-times linkage and an antigen protein linked thereto. The chaperonin-antigen protein complex as shown in Fig. 1E consists of eight fusion proteins each composed of a chaperonin subunit and an antigen protein linked thereto.

In the second examples, the chaperonin-antigen protein complex consists of a fusion protein and an independent chaperonin subunit. Herein, "independent chaperonin subunit" denotes to a chaperonin subunit that is not covalently linked to other molecules, in other words, a chaperonin subunit monomer. Figures 2A to D show some schematic illustrations of the examples. The chaperonin-antigen protein complex as shown in Fig. 2A consists of a fusion protein composed of a chaperonin subunit and an antigen protein linked thereto, and seven independent chaperonin subunits. The chaperonin-antigen protein complex as shown in Fig. 2B consists of a fusion protein composed of a chaperonin subunit 4-times linkage and an antigen protein linked thereto, and four independent chaperonin subunits. The chaperonin-antigen protein complex as shown in Fig. 2C consists of two fusion proteins each composed of a chaperonin subunit 2-times linkage and an antigen protein linked thereto, and four independent chaperonin subunits. The chaperonin-antigen protein complex as shown in Fig. 2D consists of a fusion protein composed of a chaperonin subunit and an antigen protein linked thereto, a fusion protein composed of a chaperonin subunit 2-times linkage and two antigen proteins respectively linked to the N-terminus and the C-terminus thereof, a fusion protein composed of a chaperonin subunit 3-times linkage and an antigen protein linked thereto, and two independent chaperonin subunits.

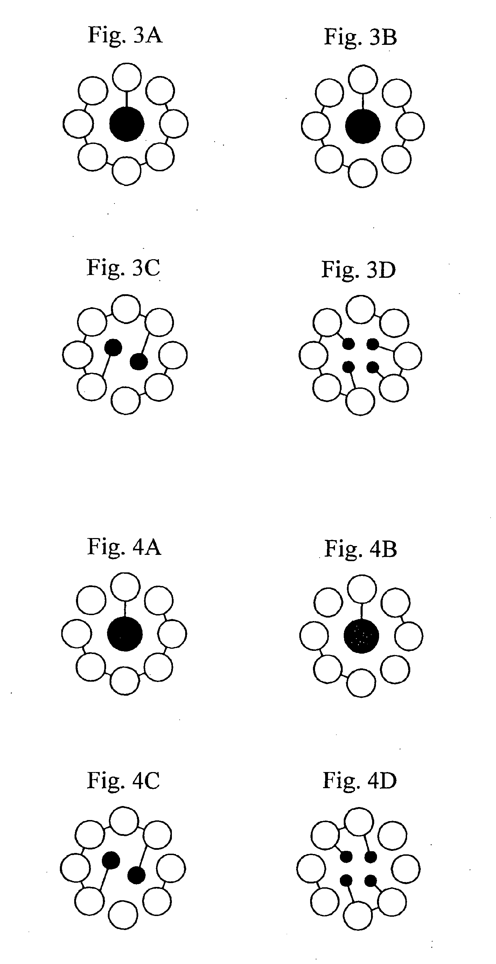

In the third examples, the chaperonin-antigen protein complex consists of a fusion protein and a chaperonin subunit linkage. Figures 3A to D show some schematic illustrations of the examples. The chaperonin-antigen protein complex as shown in Fig. 3A consists of a fusion protein composed of a chaperonin subunit and an antigen protein linked thereto, and a chaperonin subunit 7-times linkage. The chaperonin-antigen protein complex as shown in Fig. 3B consists of a fusion protein composed of a chaperonin subunit and an antigen protein linked thereto, a chaperonin subunit 3-times linkage, and a chaperonin subunit 4-times linkage. The chaperonin-antigen protein complex as shown in Fig. 3C consists of a fusion protein composed of a chaperonin subunit 5-times linkage and two antigen proteins respectively linked to the N-terminus and the C-terminus thereof, and a chaperonin subunit 3-times linkage. The chaperonin-antigen protein complex as shown in Fig. 3D consists of a fusion protein composed of a chaperonin subunit 2-times linkage and two antigen proteins respectively linked to the N-terminus and the C-terminus thereof, a fusion protein composed of a chaperonin subunit 4-times linkage and two antigen proteins respectively linked to the N-terminus and the C-terminus thereof, and a chaperonin subunit 2-times linkage.

In the fourth examples, the chaperonin-antigen protein complex consists of a fusion protein, an independent chaperonin subunit, and a chaperonin subunit linkage. Figures 4A to D show some schematic illustrations of the examples. The chaperonin-antigen protein complex as shown in Fig. 4A consists of a fusion protein composed of a chaperonin subunit and an antigen protein linked thereto, an independent chaperonin subunit, and a chaperonin subunit 6-times linkage. The chaperonin-antigen protein complex as shown in Fig. 4B consists of a fusion protein composed of a chaperonin subunit and an antigen protein linked thereto, two independent chaperonin subunits, a chaperonin subunit 2-times linkage, and a chaperonin subunit 3-times linkage. The chaperonin-antigen protein complex as shown in Fig. 4C consists of a fusion protein composed of a chaperonin subunit 5-times linkage and two antigen proteins respectively linked to the N-terminus and the C-terminus thereof, an independent chaperonin subunit, and a chaperonin subunit 2-times linkage. The chaperonin-antigen protein complex as shown in Fig. 4D consists of two fusion proteins each composed of a chaperonin subunit 2-times linkage and two antigen proteins respectively linked to the N-terminus and the C-terminus thereof, two independent chaperonin subunits, and a chaperonin subunit 2-times linkage.

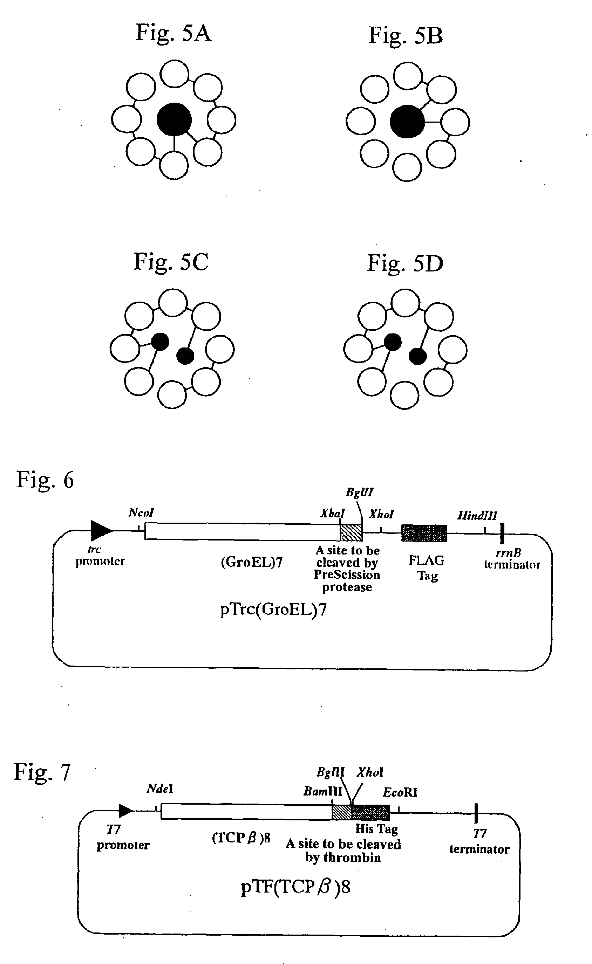

In the fifth examples, the chaperonin-antigen protein complex contains an antigen protein linked between chaperonin subunits of a chaperonin subunit linkage. Figures 5A to D show some schematic illustrations of the examples. The chaperonin-antigen protein complex as shown in Fig. 5A is a modified example of the complex as shown in Fig 1A. The chaperonin-antigen protein complex as shown in Fig. 5B is a modified example of the complex as shown in Fig 2B. The chaperonin-antigen protein complex as shown in Fig. 5C is a modified example of the complex as shown in Fig 3C. The chaperonin-antigen protein complex as shown in Fig. 5D is a modified example of the complex as shown in Fig 4C.

In the chaperonin-antigen protein complex, an amino acid sequence to be cleaved by a protease may be provided between the chaperonin subunit and the antigen protein or between the chaperonin subunits. In the fusion protein in this case, the antigen protein and the chaperonin subunit are linked via a plurality of peptide bonds. Specifically, in the chaperonin-antigen protein complex inoculated to an animal, its ring structure is gradually damaged by an attack to a linking site of the chaperonin subunits by proteases in the living body, and then the antigen protein is released by an attack to a linking site of the antigen protein and the chaperonin subunit by the proteases. Consequently, an attack by proteases to the antigen protein itself is let wait to the last, so that expression of immunogenicity of the antigen protein is prolonged. The amino acid sequence to be cleaved by a protease is not particularly limited and includes recognition sites of proteases such as PreScission protease, thrombin, Factor Xa, plasmin, and chymotrypsin.

Secondly, the case of the folding factor being a foldase will be described. Because a foldase such as PPIases is a monomer protein, the site to which an antigen protein is linked is the C-terminus and/or the N-terminus of the foldase. Therefore, a desired antigen protein may be linked to the C-terminus and/or the N-terminus of a foldase to form a fusion protein.

Further, a fusion protein composed of an antigen protein and a molecular chaperone other than chaperonins is also applicable as an immunogen.

A fusion protein used as the immunogen of the present invention does not necessarily contain the full-length of an antigen protein, and may contain a part of an antigen protein and a folding factor. Specifically, if and when a part of an antigen protein induces an immune response as well as the full-length of the antigen protein, the fusion protein composed of a part of the antigen protein and a folding factor is contained in the immunogen of the present invention. More specifically, when the immunogen of the present invention is used to give an antibody against a specific epitope of an antigen protein, a fusion protein composed of a part of the antigen protein containing the epitope and a foldase may constitute the immunogen of the present invention. It is preferable that a part of the antigen protein contains amino acid sequences of 6 or more residues of the antigen protein.

Desired antigen proteins contained in the immunogen of the present invention include all proteins having immunogenicity and are not particularly limited. Most suitable antigen proteins include an antigen protein toxic to a host cell, an antigen protein without established processes for purification, an antigen protein so liable to denaturation, an antigen protein having difficulty in preparing its transformant, and an antigen protein having an incorrect stereostructure when synthesized in a transformant cell. Although these antigen proteins are difficult to be used alone as an immunogen, a fusion protein with a folding factor is applicable as an immunogen.

Now, a method of producing the immunogen of the present invention will be described. The immunogen of the present invention contains a fusion protein composed of an antigen protein and a foldase. In order to produce the fusion protein, a gene encoding the antigen protein and a gene encoding a foldase are linked to produce a fusion gene, and then the fusion gene is expressed to synthesize the fusion protein. Specifically, a plasmid vector into which the fusion gene is integrated is introduced into a host cell to give a transformant, whereupon the fusion protein is obtained from the extract of the cultured transformant in which the fusion gene is expressed. When the folding factor is a chaperonin, an independent chaperonin subunit and/or a chaperonin subunit linkage may be required in addition to a fusion protein composed of an antigen protein and chaperonin subunits. In these cases, for example, a gene encoding an independent chaperonin subunit and/or a gene encoding a chaperonin subunit linkage may be expressed separately. A plasmid vector into which these genes are introduced may be introduced into a host cell, for example. The fusion gene, and a gene encoding an independent chaperonin subunit and/or a gene encoding a chaperonin subunit linkage may be linked polycistronically on one plasmid vector, or may be introduced respectively into two different plasmids capable of coexistence and replication in one host to co-express in one host. Further, since independent chaperonin subunits naturally occur, a gene encoding a chaperonin subunit on genomic DNA of the host cell may be transcribed and translated to provide chaperonin subunits derived from the host cell. Further, the fusion gene, an independent chaperonin subunit, and a chaperonin subunit linkage may be expressed in different host cells respectively, purified separately, and then mixed. Any antigen protein forms chaperonin-antigen protein complex when being fused with a chaperonin to constitute a fusion protein.

Generally, if and when a foreign gene being too large in size is introduced into and expressed in a host cell, an undue load is given to the host cell, resulting in a reduction in the amount of the foreign gene expression. For example, in the case of an archaeal chaperonin wherein eight chaperonin subunits form a ring structure, a fusion gene composed of eight chaperonin subunit genes linked to one another and an antigen protein gene may not be efficiently expressed. Therefore, it is preferable that a fusion protein contained in the immunogen of the present invention is as small as possible in size in respect to mass production. For that purpose, a fusion protein composed of a part, not the full-length, of an antigen protein and a folding factor is used, for example. Further, when the folding factor is a chaperonin, control of the number of chaperonin subunits contained in the fusion protein makes the fusion protein smaller in size.

Host cells into which the fusion gene or the like is introduced include, but are not particularly limited to, prokaryotes such as bacteria, and yeasts, fungi, plants, insect cells, and mammalian cells. It is more preferable to use bacteria such as Escherichia coli as a host cell because their transformants grow quite rapidly than other host cells with mass production of the fusion proteins or the like. In the case of Escherichia coli as a host cell, the fusion protein may be expressed into its cytosol, or may be expressed and secreted into its periplasm region. In order to express and secrete into its periplasm a fusion protein composed of a folding factor and an antigen protein, a secretion signal sequence region needs to be provided at the 5'-terminus of the fusion protein. Then, the fusion protein is expressed and secreted into the periplasm region because of the existence of the secretion signal sequence.

The use of a folding factor originally occurring at a membrane in a cell may further increase an expression level of the fusion protein. Examples of the folding factor include FkpA-type PPIases in FKBP-type PPIases and SurA-type PPIases in parvulin-type PPIases. In a living body, these PPIases originally occur in periplasm regions of gram-negative bacteria. Therefore, when an antigen protein is a membrane protein or the like, the protein is originally expressed out of cytoplasm, so as to be suitable to increase the expression level by being expressed as a fusion protein composed of the membrane protein and an FkpA-type PPIase or an SurA-type PPIase.

Even when other host cells are used, the fusion protein may also be expressed into its cytoplasm, or may also be expressed and secreted outside the cell. In this case, it is necessary to select a more suitable combination of a host cell and an expression vector. When the fusion protein is expressed in a mammalian cell system, for example, a promoter isolated from a genome of a mammalian cell such as mouse metallothionein promoter, or a promoter isolated from a virus growing in the cell such as baculovirus promoter and vaccinia virus 7.5K promoter is preferable as a promoter in the expression vector.

A method of introducing a plasmid vector into a host cell is not particularly limited, and may employ various known methods. Examples of transfection method include a calcium phosphate precipitation method, electoporation, liposome fusion, nuclear injection, and virus or phage infection.

It is also possible to express the fusion protein as a soluble protein in a cell-free translation system using an extract from bacteria or eukaryotes or the like (Spirin, A. S., 1991, Science 11, 2656-2664: Falcone, D. et al., 1991, Mol. Cell. Biol. 11, 2656-2664).

Methods of purification of the fusion protein constituting the immunogen of the present invention will be now described. The methods are not particularly limited and may employ conventionally known methods. The following is an example of the methods of purifying a fusion protein composed of an antigen protein and a chaperonin. A transformant in which the fusion protein is expressed is collected and disrupted to recover a cell extract. After the fusion protein in the cell extract is recovered by ammonium sulfate precipitation, the precipitated fraction is dissolved in an appropriate buffer and subjected to ion-exchange chromatography or hydrophobic chromatography to recover fractions containing the fusion protein. The fraction is concentrated by ultrafiltration and subjected to gel filtration using a buffer containing about 5-50 mM magnesium chloride and about 50-300 mM sodium chloride or potassium chloride as a developing solution, whereby a peak just after the exclusion limit is recovered to give the purified fusion protein. In the case of the fusion protein with a tag made of 6-10 histidine residues (hereinafter referred to as "histidine tag") linked to its N- or C-terminus, the fusion protein is purified more easily and efficiently by means of a metal (e.g. nickel) chelating column. Further, the fusion protein is purified rapidly and easily by immune precipitation or affinity chromatography using an antibody against the chaperonin. However, it is preferable to include ion-exchange chromatography and gel filtration to recover only the fusion protein having a ring structure in the method. In the case of the chaperonin having heat resistance, heat treatment of the extract from the host at 60 to 80°C precipitates and removes the majority of residual proteins derived from the host, thereby purify the fusion protein more simply. Even if the antigen protein itself is not heat-resistant, the fusion protein is not thermally denatured because it is held inside of the chaperonin.

The form of the fusion protein obtained by any method of purification described above is observed under a transmission electron microscope. When the desired antigen protein is accommodated inside of the chaperonin ring, a ring structure with an external diameter of about 14-16 nm, unique to chaperonins, is observed. When the ring structure of the fusion protein is unstable, a fusion protein having the ring structure is efficiently recovered in the presence of magnesium and ATP in the purification process.

When the desired antigen protein is a membrane-binding protein or a transmembrane protein, the fusion protein is purified and treated with an nonionic detergent whose hydrophobic alkyl grope is octyl (C8) to dodecyl (C12), whereby the antigen protein easily form the correct structure inside of the chaperonin because the resulting micelle has the diameter substantially the same as the thickness of the biomembrane. Examples of the nonionic detergent include β-octylglucoside, Triton X100, Nonidet P-400 and Tween20.

When a chaperonin-antigen protein complex contains not only a fusion protein but also an independent chaperonin subunit or a chaperonin subunit linkage, they may be expressed in different hosts, separately purified, and then mixed to give the complex.

Although the immunogen of the present invention induces high immune responses when being inoculated to an animal directly, a composition for immunological use prepared by mixing the immunogen with an adjuvant has higher immunogenicity. Consequently, the composition for immunological use produces an antibody more efficiently. Examples of the adjuvant include complete Freund's adjuvant, incomplete Freund's adjuvant, aluminium adjuvant, and pertussis adjuvant.

Methods of producing antibodies using the immunogen of the present invention will be described.

Methods of immunizing an animal with the immunogen of the present invention may employ commonly known methods for immunization. For example, the immunogen of the present invention is administered intravenously, intradennally, hypodermicly, intramuscularly or intraperitoneally, to animals such as mice, rats, hamsters, marmots, rabbits, dogs, cats, sheep, goats and horses by injection or the like, thereby immunizing the animals with the antigen protein to be targeted at. A polyclonal antibody is obtained from a serum that is obtained from an animal administered with the immunogen of the present invention by the above-described method. The polyclonal antibody is purified from the serum by means of protein A affinity chromatography or the like.

A monoclonal antibody is produced from a single clone that is isolated by screening of a hybridoma secreting an antibody bound to a target antigen protein from the group of hybridomas produced by cell fusion of an antibody-producing cell (spleen cell) with a myeloma cell. This method, which is called "cell fusion method", is commonly used for preparation of monoclonal antibodies (Sakuji Toyama and Tamie Ando, "Experiment manual for monoclonal antibodies", Kodansha, 1987).

More specifically, the immunogen of the present invention is administered to an animal to be immunized by the above-described method, whereby an immunocyte to produce a monoclonal antibody such as a spleen cell after immunization is obtained. Then, a clone producing an antibody recognizing a desired epitope is selected from hybridomas of the immunocyte and an animal myeloma, and cultured to produce a target monoclonal antibody. The myeloma, as a parent cell for the cell fusion with the immunocyte, may be appropriately selected from the conventionally known myelomas and includes SP2/0-Ag14, P3-NS1-1-Ag4-1 and MCP11-45, 6.TG1.7 (derived from mice); 210.RCYAg1.2.3 (derived from rats); SKO-007 and GM15006TG-A12 (derived from human).

The above-described cell fusion of the immunocyte and these myelomas is carried out by commonly known methods such as the method of Koehler and Milstein (Koehler, G. and Milstein, C., Nature, 256, 495 (1975)). More specifically, the cell fusion is carried out in a common medium to which assisting agents such as dimethyl sulfoxide are added, if necessary, to improve fusion efficiency, in the presence of the commonly known fusion promoting agent such as polyethylene glycol (PEG) and Sendai virus (HVJ), thereby preparing a group of hybridomas.

A desired hybridoma is separated from the group of hybridomas cultured in a common selection medium such as HAT (hypoxanthine, aminopterin and thymidine) medium. More specifically, the desired hybridoma is separated from the group of hybridomas cultured in the selection medium for enough time to kill cells except the desired hybridoma. The hybridoma obtained in this way is subjected to searching for a desired monoclonal antibody and cloning by a common limiting dilution method.

In order to search a strain producing a desired monoclonal antibody from the obtained hybridomas, common methods such as ELISA method, plaque method, spot method, agglutination method, Ouchterlony method and RIA method may be used.

Hybridomas are sub-cultured in a common medium and also preserved in liquid nitrogen for a long time.

A desired monoclonal antibody is obtained from a hybridoma by methods such as a method including steps of culturing the hybridoma according to a common manner and obtaining the desired monoclonal antibody from the supernatant of the culture, and a method including steps of administering the hybridoma to an animal compatible to the hybridoma to grow the hybridoma and obtaining the desired monoclonal antibody from its ascites. The monoclonal antibody obtained in this way is further purified by a common method such as salting out, gelfiltration and affinity chromatography. The monoclonal antibody obtained in this way specifically recognizes an epitope in the fusion protein subjected to the immunization.

Recently, techniques of producing a monoclonal antibody using a cloned antibody gene from an antibody-producing cell (spleen cell) have been developed. Examples of the technique include phage display method whereby a desired antibody gene is easily screened by expression of the antibody on the surface of the phage transformed with a gene encoding the antibody from an antibody-producing cell.

One of the methods of preparing a desired monoclonal antibody by phage display method is disclosed in Japanese laid-open patent H07-502167. Specifically, a region corresponding to Fab is amplified by RT-PCR method using mRNA isolated from an antibody-producing cell of an animal immunized with the fusion protein and primers specific to a region between complementarity determining regions (CDR), and is introduced into a phagemid vector to give an antibody library maintaining a high degree of diversity. Clones specifically producing Fabs in the presence of helper phages are concentrated and screened using the antigen for immunization, so that a desired Fab is expressed in Escherichia coli in a large amount. After its immunological activity is examined well, its whole-type IgG is expressed in a cultured cell with an expression vector of Fc part with which the desired Fab is combined.

The immunogen of the present invention using serotonin receptor as an antigen protein will be described by the following example. However, this invention is not limited to the specific antigen protein. It is easy for persons ordinarily skilled in the art to obtain advantages of the present invention without unnecessary experiments by replacing the serotonin receptor with other desired proteins.

Serotonin (also referred to as 5-hydroxy-tryptamine), a potent, naturally occurring neurotransmitter that exerts its effects via cell surface receptors found on serotonergic neurons. It has been revealed that serotonin plays a significant role in functioning of mammalian body, both in the central nervous system (CNS) and in peripheral system. It has been revealed that serotonergic neurons, which originate in the brain stem, form a very diffuse system that projects to most areas of the brain and spinal cord. With such a diffuse system, it is considered that serotonin is involved in physiological responses and expression of diseases that originate in the CNS, namely, a number of behaviors such as sleeping, eating, perceiving pain, controlling body temperature, controlling blood pressure, depression, and schizophrenia.

Serotonin also plays a significant role in peripheral systems as well. For example, multiple serotonins are found in gastrointestinal system, and mediate a variety of contractile, secretory, and electrophysiologic effects in this system. Another example highly sensitive to serotonin is cardiovascular systems.

Serotonin induces its effects on cellular physiology by binding to a receptor on the cell surface. At present, it is confirmed that multiple types of serotonin receptors occur. At least four major families of serotonin receptors (5-HT1R-4R) are known, each family containing 8 to 10 receptors that have been classified based on their pharmacological and/or structural differences. Each family consists of one or more types called 5-HT1aR, 5-HT1bR, 5-HT1dR and 5-HT1eR. Further, it is known that various subtypes such as HT1dαR and 5-HT1dβR known within certain serotonin receptor types.

Existence of multiple, structurally distinct serotonin receptors provides the possibility that subtype-selective pharmacologic agents can be produced. Since individual receptor subtypes may function to affect specific actions of the different parts of the central and peripheral nerve system, the development of such agents result in new and increasingly selective therapeutic agents with fewer side effects. As an example, in certain blood vessels, stimulation of 5-HT1 receptors on the endothelial cells induces vasodilation while stimulation of 5-HT2 receptors on the smooth muscle cells induces vasoconstriction. Pharmacological and clinical knowledge of the various serotonin receptor types is described in detail in, for example, Glennon RA. et al. Neuroscience and Behavioral Reviews, 14(1), page 35 (1990).

Given the broad distribution of serotonin within the body, tremendous interest in developing drugs that affect serotonergic systems exists. Especially, receptor-specific agonists and antagonists are of interest for therapeutic agents for a wide range disorders, including anxiety, depression, hypertension, migraine, compulsive disorders, and cancer chemotherapy-induced vomiting. An antibody against a serotonin receptor is not only useful for research but also a candidate for receptor-specific agonists and antagonists as a drug.

5-HT1aR is one of the serotonin receptors and a seven transmembrane protein, and is known as an antigen protein that is hard to obtain its recombinant protein and an excellent antibody thereagainst.

When a complex composed of a folding factor and 5-HT1aR is used as the immunogen of the present invention, the complex induces a high immune response in an immunized animal. Especially, it is preferable that a fusion protein composed of a FKBP-type PPIase or a chaperonin and 5-HT1aR is used as an immunogen. It is preferable that Escherichia coli chaperonin GroEL or archaeal chaperonin TCP is used as a chaperonin. In this case, the full-length of serotonin receptor 5-HT1aR or a part consisting of 6 or more residues thereof is used as an antigen protein.

It is obvious that, in any case, the immunogen of the present invention is effective in increasing an immune response against 5-HT1aR because 5-HT1aR induces a specific immune response only when being fused with a folding factor.

According to the present invention, any antigen protein induces a higher immune response by using a fusion protein composed of the antigen protein and a folding factor than the antigen protein alone.

### EXAMPLES

Hereinafter, the present invention will be described in more detail by reference to Examples. However, the present invention is not limited to the Examples.

In the following Examples, many techniques well known by and available to skilled persons in the field of molecular biology, protein chemistry or immunology are used, but are not necessarily described in detail. Reagents such as enzymes were obtained on the market and used in accordance with the protocols provided by the distributors. Other experimental techniques employed techniques described in "Sambrook et al., Molecular Cloning: A Laboratory Manual, CHS Press, 1989" or the like.

### Example 1

### (An immunogen containing a complex composed of Escherichia coli chaperonin GroEL and an antigen protein)

### 1. Construction of a vector to express a fusion protein with GroEL

The nucleotide sequence of Escherichia coli chaperonin GroEL gene, which has been known, is shown in SEQ ID No. 1. Oligonucleotides as shown in SEQ ID Nos. 2 and 3 were prepared based on the information of this nucleotide sequence. PCR using the genomic DNA of Escherichia coli HMS174 (Novagen) as a template and oligonucleotides as shown in SEQ ID Nos. 2 and 3 as a primer set was carried out, whereby a DNA fragment containing GroEL gene was amplified. Herein, an SpeI site and an XbaI site derived from the primers were introduced into each terminus of the amplified DNA fragment. The amplified DNA fragment was introduced into pT7BlueT vector (Novagen) by TA cloning. The nucleotide sequence of the introduced DNA fragment was determined, and confirmed to be the same as the nucleotide sequence as shown in SEQ ID No.1.

Meanwhile, a synthetic DNA as shown in SEQ ID No. 4 and a synthetic DNA as shown in SEQ ID No. 5 that are complementary nucleotide sequences were annealed to make a double-stranded DNA. The double-stranded DNA had an XbaI site, a nucleotide sequence encoding a PreScission protease recognition site, and a BglII site in order of mention. The double-stranded DNA was recovered by XbaI and BglII treatment, and introduced into pTrc99ALTCF (Pharmacia) treated in advance with XbaI and BglII to give a plasmid pTRC99-PS. That is, pTRC99-PS is an expression plasmid containing a nucleotide sequence encoding a site to be cleaved by PreScission protease between the Xba I site and the Bgl II site.

Then, in order to recover a DNA fragment containing GroEL gene, the pT7BlueT vector into which the amplified DNA was introduced was treated with restriction enzymes SpeI and XbaI. The isolated DNA fragment was introduced into pTRC99-PS treated in advance with XbaI to give pTrcGroEL vector.

Next, pTrcGroEL vector was treated with XbaI, and connected again to the isolated DNA fragment containing GroEL gene obtained by treatment of pT7BlueT vector into which the above-described amplified DNA fragment with restriction enzymes SpeI and XbaI to give a vector pTrc(GroEL)2 expressing a GroEL 2-times linkage. Repetition of the same procedure prepared a vector pTrc(GroEL)7 expressing a GroEL 7-times linkage. Figure 6 shows a constitution of pTrc(GroEL)7. Specifically, pTrc(GroEL)7 has a trc promoter and a rbb terminator. Further, pTrc(GroEL)7 has a GroEL 7-times linkage gene composed of seven GroEL genes arranged in tandem, a nucleotide sequence encoding a PreScission protease recognition amino acid sequence, and a nucleotide sequence encoding the amino acid sequence of FLAG at the downstream of the trc promoter.

On the other hand, human serotonin receptor 5-HT1aR was employed as a desired antigen protein. The nucleotide sequence of 5-HT1aR gene, which was known, was shown in SEQ ID No. 6. Oligonucleotides as shown in SEQ ID Nos. 7 and 8 were prepared based on the information of this nucleotide sequence. PCR using a human brain cDNA library (Takara Bio) as a template and oligonucleotides as shown in SEQ ID Nos. 7 and 8 as a primer set was carried out, whereby an DNA fragment containing 5-HT1aR gene was amplified. Herein, a BglII site and an XhoI site derived from the primers were introduced to each terminus of the amplified DNA fragment. The amplified DNA fragment was introduced into pT7BlueT vector (Novagen) by TA cloning. The nucleotide sequence of the introduced DNA fragment was determined, and confirmed to be the same as the nucleotide sequence as shown in SEQ ID No. 6. Then, the amplified DNA fragment was introduced into pTrc(GroEL)7 treated in advance with BglII and XhoI to give a vector pTrc(GrOEL)7• 5HT1AR expressing a fusion protein composed of a GroEL 7-times linkage and 5-HT1aR. Meanwhile, an expression vector pTHT1AR into which 5-HT1AR gene alone was introduced instead of a fusion protein gene was prepared for a control experiment.

### 2. Expression of a fusion protein composed of a GroEL 7-times linkage and serotonin receptor 5-HT1aR

Escherichia coli strain BL21(DE3) (Novagen) was transformed with the obtained expression vector pTrc(GroEL)7 - 5HT1AR to give a transformant expressing a fusion protein composed of a GroEL 7-times linkage and serotonin receptor 5-HT1aR.

Twenty to twenty-five colonies of the obtained transformant were inoculated to 500mL of 2xTY medium (16 g/L Yeast extract, 20 g/L Bacto-tripton, 15 g/L NaCl, 0.1 mg/mL Ampicillin), and cultured at 110 rpm for 24 hours at 25°C to give a cultured broth. The cultured broth was centrifuged at 10000 rpm for 10 minutes, so that the transformant cells were harvested.

The harvested cells were suspended in FLAG-binding buffer (50 mM Tris-HCl pH 7.5, 150 mM NaCl, 5 mM MgCl, 1 mM EDTA), and then disrupted by sonication. The disrupted cell suspension was centrifuged at 30,000 rpm for 1 hour to give its supernatant that is a soluble fraction. Meanwhile, the same procedure was carried out using Escherichia coli strain BL21(DE3) harboring a vector pTHT1AR for a control experiment.

When supernatants and precipitate fractions of the Escherichia coli extracts were analyzed by SDS-PAGE, only from the soluble fraction of the extracted sample of Escherichia coli cells harboring the expression vector pTrc(GroEL)7 • 5HT1AR, a band at the position corresponding to the molecular weight of the fusion protein was detected. On the other hand, the antigen proteins were not detected from the soluble fraction of the extract sample of Escherichia coli cells harboring pTHTIAR as a control. although a weak band was detected from the insoluble fraction of the extract sample of Escherichia coli cells harboring pTHT1AR. From these results, it was found that 5-HT1aR gene alone was not expressed in the Escherichia coli soluble fraction, but expressed as a soluble protein when constituting a fusion protein with a GroEL 7-times linkage.

Then, the obtained supernatant was salted out, whereupon the precipitated fusion protein was collected. The precipitate was dissolved in 50 mM Tris-HCl (pH 7.5), and then dialyzed against 50 mM Tris-HCl (pH 7.5) containing 5 mM MgCl₂. After the dialysis, the dialysate was subjected to ion-exchange chromatography using DEAE-Sepharose column (Amersham Bioscience) and TSKgel Super Q-5PW column (Tosoh), and to gel filtration using Superose 6 column (Amersham Bioscience), whereby the fusion protein was purified from the dialysate. As a result, a fusion protein composed of a GroEL 7-times linkage and 5-HT1aR was obtained.

### 3. Confirmation of the structure of the fusion protein by electron microscopy

The structures of the obtained fusion protein composed of a GroEL 7-times linkage and 5-HT1aR was confirmed under transmission electron microscope (TEM) according to the following procedures. Ten µL of purified preparation of the fusion protein with an adjusted concentration of about 0.05 mg/mL was fixed on a carbon-coated formvar membrane on copper grid, stained with 2% uranyl acetate, and then observed at 75 kV of accelerating voltage. The result was that the fusion protein had a ring structure unique to chaperonins and that the 5-HT1aR was accommodated one-by-one in the GroEL ring. Consequently, it was confirmed that the fusion protein formed a complex having a ring structure as well as native GroEL.

### 4. Digestion with a protease

It was examined whether the PreScission protease recognition sequence between the GroEL 7-times linkage and the 5-HT1aR in the obtained fusion protein was recognized by PreScission protease. The obtained fusion protein was incubated in the presence of 2.5 M urea, and then dialyzed against 50 mM potassium phosphate (pH 7.0). The solution after dialysis was treated with PreScission protease with all day and night incubation at 15°C. As a result of analysis for the reaction solution by SDS-PAGE, a band corresponding to the 5-HT1aR was detected, and therefore the fusion protein was cleaved by PreScission protease. On the other hand, when the obtained fusion protein was not treated with urea, a band corresponding to the 5-HT1aR was not detected, and therefore the fusion protein was not cleaved by PreScission protease. From these results, it was thought that the 5-HT1aR was protected by the GroEL because the fusion protein formed a complex composed of GroEL and 5-HT1aR.

### 5. Evaluation as an immunogen

The obtained fusion protein composed of a GroEL 7-times linkage and 5HT1aR was evaluated for its ability of inducing an immune response against 5-HT1aR according to the following procedures. A composition for immunological use was prepared by mixture of the obtained fusion protein and incomplete Freund's adjuvant and injected into rabbits hypodermicly. As it is considered that an antibody titer usually increased 7 to 10 days after the sensitization, the serum was sampled on the 3rd, 5th, 7th, and 10th days after the immunization and then analyzed by the following ELISA method in order to evaluate strength of the immune response. 5-HT1aR solution (Eurosecreen S.A.) was added to a 96-well plate and incubated for 3 hours at 30°C, whereby 5-HT1aR was immobilized on the plate. After being washed with PBS, the plate was blocked with 1% BSA in PBS. The sampled serum was serially diluted with 1% BSA/PBS containing 0.05% Tween 20. The diluted serum was added to the well coated with 5-HT1aR, and then incubated for 1 hour at room temperature. After being washed with PBS containing 0.05% Tween 20, the plate, to which peroxidase-conjugated anti-rabbit IgG antibody (Vector) was added, was incubated for 1 hour at room temperature. One hundred microliters of peroxidase substrate (ABTS) solution (KPL) was added to each well, and then its absorbance at 410 nm was determined. The end point of ELISA titers for the serum was designed to be the serum dilution producing an absorbance value 2 SD greater than the average of the control rabbit. A control experiment was carried out by using rabbits injected with 5-HT1aR alone. The experiments showed that the use of a fusion protein composed of a chaperonin and 5-HT1aR as an immunogen induced a higher immune response for rabbits than the use of 5-HT1aR alone (TABLE 1).

As observed above, it was revealed that the 5-HT1aR became soluble and was made available as an immunogen when being fused with a GroEL 7-times linkage to give a fusion protein, and that an immunogen containing the fusion protein composed of a GroEL 7-times linkage and a 5-HT1aR was also effective to strengthen immune responses against the 5-HT1aR.

**TABLE 1. Results of ELISA using sera of rabbits immunized by 5-HT1aR alone or 5-HT1aR fused with a GroEL 7-times linkage**

| Day of blood sampling | Fusion protein ELISA titer^{(b)} | 5-HT1aR ELISA titer | Control^{(a)} ELISA titer |
|---|---|---|---|
| after 3 days | 1/10 | <1/10 | <1/10 |
| after 5 days | 1/500 | <1/10 | <1/10 |
| after 7 days | 1/2000 | 1/10 | <1/10 |
| after 10 days | 1/4000 | 1/10 | <1/10 |

| | | | |
|---|---|---|---|
| (a) Only incomplete Freund's adjuvant was injected into a rabbit in the control experiment. | | | |
| (b) The titers are expressed as dilution ratio of serum. | | | |

### Example 2

### (An immunogen containing a complex composed of archaeal chaperonin TCP and an antigen protein)

### 1. Construction of a vector to express a fusion protein with a TCPβ 8-times linkage

The nucleotide sequence of chaperonin β subunit (TCPβ) gene of Thermococcus sp. strain KS-1 (JCM No. 11816), which has been known, was shown in SEQ ID No. 9. Oligonucleotides as shown in SEQ ID Nos. 10 and 11 were prepared based on the information of this nucleotide sequence. PCR using the genomic DNA of Thermococcus sp. strain KS-1 (JCM No. 11816) as a template and oligonucleotides as shown in SEQ ID Nos. 10 and 11 as a primer set was carried out, whereby a DNA fragment containing TCPβ gene was amplified. Herein, a BglII site and a BamHI site derived from the primers were introduced into each terminus of the amplified DNA fragment. The amplified DNA fragment was introduced into pT7BlueT vector (Novagen) by TA cloning to give pT7(TCPβ). The nucleotide sequence of the introduced DNA fragment was determined, and confirmed to be the same as the nucleotide sequence as shown in SEQ ID No. 9. Then, pT7(TCPβ) was treated with BglII and BamHI, and a DNA fragment containing containing the TCPβ gene was recovered. The DNA fragments were linked to each other by means of T4 DNA ligase, and then separated by means of electrophoresis for the reaction solution, whereby (TCPβ)2 gene consisting of two TCPβ genes linked in the same direction was recovered. (TCPβ)2 gene was introduced into a plasmid pKF3 (Takara Bio) treated in advance with BglII and BamHI to give a vector pKF(TCPβ)2. In the same way, pKF(TCPβ)2 was treated with BglII and BamHI to give a DNA fragment containing (TCPβ)2 gene. The DNA fragments were linked to each other by means of T4 DNA ligase, and then separated by means of electrophoresis for the reaction solution, whereby (TCPβ)4 gene consisting of four TCPβ genes linked in the same direction was recovered. The (TCPβ)4 gene was introduced into a plasmid pKF3 treated in advance with BglII and BamHI to give a vector pKF(TCPβ)4. In the same way, pKF(TCPβ)4 was treated with BglII and BamHI to give a DNA fragment containing (TCPβ)4 gene. The DNA fragments were linked to each other by means of T4 DNA ligase, and then separated by means of electrophoresis for the reaction solution, whereby (TCPβ)8 gene consisting of eight TCPβ genes linked in the same direction was recovered.

Meanwhile, a synthetic DNA as shown in SEQ ID No. 12 and a synthetic DNA as shown in SEQ ID No. 13 that are complementary nucleotide sequences were annealed to make a double-stranded DNA. The double-stranded DNA had an NdeI site, a BamHI site, a nucleotide sequence encoding a thrombin recognition site, a BglII site, an XhoI site, a nucleotide sequence encoding six sequential histidines (His tag), and an EcoRI site in order of mention.

The double-stranded DNA was treated with NdeI and EcoRI, and introduced into a plasmid pET21d (Novagen) treated in advance with NdeI and EcoR to give a plasmid pTF. Further, the (TCPβ)8 gene was introduced into the plasmid pTF treated in advance with BamHI to give a plasmid pTCP8. Figure 7 shows a constitution of pTCP8. Specifically, pTCP8 has a T7 promoter and a T7 terminator. Further, pTCP8 has a TCPβ 8-times linkage ((TCPβ)8) gene composed of eight TCPβ genes arranged in tandem, a nucleotide sequence encoding a thrombin recognition site, and a nucleotide sequence encoding an amino acid sequence of His tag at the downstream of the T7 promoter.

On the other hand, human serotonin receptor 5-HT1aR was employed as a desired antigen protein as well as Example 1. The amplified DNA fragment containing 5-HT1aR gene obtained in Example 1 was treated with BgIII and XhoI, and introduced into pTCP treated in advance with BamHI to give a vector pTCP8 • 5HT1AR to express a fusion protein composed of a TCPβ 8-times linkage and 5-HT1aR. That is, a fusion protein composed of a TCPβ 8-times linkage linked to 5HT1aR via a thrombin recognition amino acid sequence is obtained by using pTCP8• 5HT1AR. Meanwhile, an expression vector pTHT1AR into which 5-HT1AR gene alone was introduced was used as a control as well as Example 1.

### 2. Expression of a fusion protein composed of a TCPβ 8-times linkage and serotonin receptor 5-HT1aR

The obtained expression vector pTCP8•5HT1AR was introduced into Escherichia coli strain BL21 (DE3) to give a transformant expressing a fusion protein composed of a TCPβ 8-times linkage and serotonin receptor 5-HT1aR.

Ten colonies of the obtained transformants were inoculated to 500 mL of 2xTY medium, and then cultured at 35°C. When cell density of the medium reached OD600 of 0.6 to 1.0, IPTG was added to the medium to induce the protein expression. The transformants were cultured for another 10 hours, and were harvested as well as Example 1.

The harvested cells were suspended in His tag-binding buffer (20 mM sodium phosphate pH 7.4, 500 mM NaCl, 10 mM imidazole), and then disrupted by sonication. The disrupted cell suspension was centrifuged at 10,000 rpm for 1 hour to give its supernatant which is a soluble fraction. Meanwhile, the same procedure was carried our using Escherichia coli strain BL21(DE3) harboring vector pTHT1AR for a control experiment.

When supernatants and precipitate fractions of the Escherichia coli extracts were analyzed by SDS-PAGE, only from the soluble fraction of the extracted sample of Escherichia cells harboring the expression vector pTCP8 • SHT1AR, a band at the position corresponding to the molecular weight of the fusion protein was detected. On the other hand, the antigen proteins were not detected from the soluble fraction of the extract sample of Escherichia coli cells harboring pTHT1AR as a control, although a weak band was detected from the insoluble fraction of the extract sample of Escherichia coli cells harboring pTHT1AR. From these results, it was found that 5-HT1aR gene alone was not expressed in the Escherichia coli soluble fraction, but expressed as a soluble protein when constituting a fusion protein with a TCPβ 8-times linkage.

A part of the obtained supernatant was applied onto HiTrap chelating 5 mL column (Amersham Bioscience). The column was washed with the His tag-binding buffer, whereupon the fusion protein was eluted by a His tag-elution buffer (20 mM sodium phosphate pH 7.4, 500 mM NaCl, 500 mM imidazole). The obtained fraction was dialyzed against a buffer containing 50 mM MgCl₂, 0.2 mM ATP, and 150 mM NaCl. The resulting dialysate was subjected to gel filtration chromatography using TSKGelG4000 (Tosoh) with the same buffer as a developing solution to recover a peak just after the exclusion limit, whereby the purified fusion protein composed of a TCPβ 8-times linkage and 5-HT1aR was obtained.

### 3. Confirmation of the structure of the fusion protein by electron microscopy

The structures of the obtained fusion protein composed of a TCPβ 8-times linkage and 5-HT1aR was confirmed under transmission electron microscope as well as Example 1. The result was that the fusion protein had a ring structure unique to chaperonins and that the 5-HT1aR was accommodated one-by-one in the TCP ring. Consequently, it was confirmed that the fusion protein formed a complex having a ring structure as well as native TCP.

### 4. Digestion with a protease

It was examined whether the thrombin recognition sequence between the TCPβ 8-times linkage and the 5-HT1aR in the obtained fusion protein was recognized by thrombin. The obtained fusion protein was incubated in the presence of 10 mM EDTA, and then dialyzed against 50 mM potassium phosphate (pH 7.0). The solution after dialysis was treated with thrombin with all day and night incubation at 15°C. As a result of analysis for the reaction solution by SDS-PAGE, a band corresponding to 5-HT1aR was detected, and therefore the fusion protein was cleaved by thrombin. On the other hand, when the obtained fusion protein was not treated with EDTA, a band corresponding to the 5-HT1aR was not detected, and therefore the fusion protein was not cleaved by thrombin. From these results, it was thought that the 5-HT1aR was protected by the TCP because the fusion protein formed a complex composed of TCP and 5-HT1aR.

### 5. Evaluation as an immunogen

The obtained fusion protein composed of a TCPβ 8-times linkage and SHT1aR was evaluated for its ability of inducing an immune response against 5-HT1aR as well as Example 1. The experiments showed that the use of a fusion protein composed of a TCPβ 8-times linkage and 5-HT1aR as an immunogen induced a higher immune response for rabbits than the use of 5-HT1aR alone (TABLE 2). As observed above, it was revealed that an immunogen containing the fusion protein composed of a TCP 8-times linkage and 5-HT1aR prepared in this Example was also effective to strengthen immune responses against 5-HT1aR.

**TABLE 2. Results of ELISA using sera of rabbits immunized by 5-HT1aR alone or 5-HT1aR fused with a TCPβ 8-times linkage**

| Day of blood sampling | Fusion protein ELISA titer^{(b)} | 5-HT1aR ELISA titer | Control^{(a)} ELISA titer |
|---|---|---|---|
| after 3 days | 1/10 | <1/10 | <1/10 |
| after 5 days | 1/500 | <1/10 | <1/10 |
| after 7 days | 1/2000 | 1/10 | <1/10 |
| after 10 days | 1/4000 | 1/10 | <1/10 |

| | | | |
|---|---|---|---|
| (a) Only incomplete Freund's adjuvant was injected into a rabbit in the control experiment. | | | |
| (b) The titers are expressed as dilution ratio of serum. | | | |

### Example 3

### (Construction of a vector to express a fusion protein composed of a short-type FKBP-type PPIase derived from extremely thermophilic archaeum Thermococcus sp. KS-1 (TcFKBP18) and a target protein)

### 1. Construction of a vector to express a fusion protein with TcFKBP18

The nucleotide sequence of short-type FKBP-type PPIase derived from extremely thermophilic archaeum Thermococcus sp. KS-1 (TcFKBP18) (Ideno et al. Biochem. J. 357, 465-, 2001), which has been known, was shown in SEQ ID No. 14. Oligonucleotides as shown in SEQ ID Nos. 15 and 16 were prepared based on the information of this nucleotide sequence. PCR using a plasmid pEFE1-3 (Iida et al., Gene, 222, 249-, 1998) containing TcFKBP18 as a template and oligonucleotides as shown in SEQ ID Nos. 15 and 16 as a primer set was carried out, whereby a DNA fragment containing TcFKBP18 gene was amplified. Herein, an NcoI site and an SpeI site derived from the primers were introduced into each terminus of the amplified DNA fragment. The amplified DNA fragment was introduced into pT7BlueT vector by TA cloning. The nucleotide sequence of the introduced DNA fragment was determined, and confirmed to be the same as the nucleotide sequence as shown in SEQ ID No. 14.

Meanwhile, oligonucleotides as shown in SEQ ID Nos. 17 and 18 were synthesized. These oligonucleotides have complementary nucleotide sequences to each other. These oligpnucleotides were annealed to give a double-stranded DNA fragment Throm-F2. Throm-F2 contains an SpeI site, a nucleotide sequence encoding an amino acid sequence to be cleaved by thrombin, a BamHI site, an Ndel site, and an EcoRI site.

The obtained DNA fragment containing the TcFKBP18 gene and Throm-F2 fragment were treated with NcoI and SpeI, and SpeI and EcoRI, respectively, and each of the fragments were recovered. Both of the recovered fragments were introduced into a plasmid pET21d (Novagen) treated in advance with SpeI and EcoRI in such a direction as TcFKBP18 gene-Throm-F2 fragment to give a plasmid TcFKfusion2 which expresses a fusion protein composed of TcFKBP18 and a target protein. Figure 8 shows a constitution of TcFKfusion2. Specifically, TcFKfusion2 has a T7 promoter and a T7 terminator. Further, TcFKfusion2 has a TcFKBP18 gene, a nucleotide sequence encoding a thrombin recognition amino acid sequence, and a multi-cloning site derived from pET21d at the downstream of the T7 promoter. That is, a fusion protein composed of a TcFKBP18 and a target antigen protein is obtained by introduction of a target antigen protein gene into the multi-cloning site of the TcFKfusion2.

On the other hand, human serotonin receptor 5-HT1aR was employed as a desired antigen protein as well as Examples 1 and 2. However, oligonucleotides as shown in SEQ ID Nos. 19 and 20 were used as a primer set for PCR to obtain 5-HT1aR gene. Herein, an NdeI site was introduced into the 5'-terminus of the SHT1aR gene, and an SacI site and nucleotide sequence encoding His tag were introduced into the 3'-terminus of the 5HT1aR gene. The amplified DNA fragment was introduced into pT7BlueT vector by TA cloning. The nucleotide sequence of the introduced DNA fragment was determined, and confirmed to be the same as the nucleotide sequence as shown in SEQ ID No. 6. The amplified DNA fragment was introduced into TcFKfusion2 treated in advance with NdeI and SacI to give a vector TcFKfusion2•5HT1 aR to express a fusion protein composed of TcFKBP18 and 5-HT1aR.

### 2. Expression of a fusion protein composed of TcFKBP18 and serotonin receptor 5-HT1aR

The obtained expression vector TcFKfusion2 • 5HT1aR was introduced into Escherichia coli strain BL21 (DE3) to give a transformant expressing a fusion protein composed of TcFKBP18 and serotonin receptor 5-HT1aR.

One to two loops of the obtained transformants were inoculated to 700mL of 2xTY medium, and then cultured at 35°C for 24 hours at 110 rpm to give a cultured broth. The cultured broth was centrifuged at 10000 rpm for 10 minutes, so that the transformant cells were harvested. The harvested cells were suspended in His-tag-binding buffer (20 mM sodium phosphate pH 7.4, 500 mM NaCl, 10 mM imidazole), and then disrupted by sonication. The disrupted cell suspension was centrifuged at 10,000 rpm for 10 minutes to give its supernatant that is a soluble fraction. When the supernatant was analyzed by SDS-PAGE, a band was detected at the position corresponding to the molecular weight of the fusion protein composed of TcFKBP18 and 5-HT1aR. Further, Western Blotting using anti-serotonin receptor antibody (Cosmobio) confirmed that the band contained 5HT1aR.

A part of the obtained supernatant was applied onto HiTrap chelating 5 mL column (Amersham Bioscience). The column was washed with the His tag-binding buffer, whereupon the fusion protein composed of TcFKBP18 and 5-HT1aR was eluted by the His tag-elution buffer.

### 3. Evaluation as an immunogen

The obtained fusion protein composed of TcFKBP18 and 5-HT1aR was evaluated for its ability of inducing an immune response against 5-HT1aR as well as Example 1. The experiments showed that the use of a fusion protein composed of TcFKBP18 and 5-HT1aR as an immunogen induced a higher immune response for rabbits than the use of 5-HT1aR alone (TABLE 3). As observed above, it was revealed that an immunogen containing the fusion protein composed of TcFKBP18 and 5-HT1aR prepared in this Example was also effective to strengthen immune responses against 5-HT1aR.

**TABLE 3. Results of ELISA using sera of rabbits immunized by 5-HT1aR alone or 5-HT1aR fused with TcFKBP18**

| Day of blood sampling | Fusion protein ELISA titer^{(b)} | 5-HT1aR ELISA titer | Control^{(a)} ELISA titer |
|---|---|---|---|
| after 3 days | 1/10 | <1/10 | <1/10 |
| after 5 days | 1/500 | <1/10 | <1/10 |
| after 7 days | 1/2000 | 1/10 | <1/10 |
| after 10 days | 1/4000 | 1/10 | <1/10 |

| | | | |
|---|---|---|---|
| (a) Only incomplete Freund's adjuvant was injected into a rabbit in the control experiment. | | | |
| (b) The titers are expressed as dilution ratio of serum. | | | |

### INDUSTRIAL APPLICABILITY

According to the immunogen of the present invention, a sufficient immune response is induced even from an antigen protein which has been considered unavailable to induce any effective immune responses for various reasons. Therefore, an antibody against a desired antigen is produced more certainly.

## Claims

1. An immunogen for inducing an immune response to a desired antigen protein, the immunogen comprising:
a fusion protein composed of one selected from the full-length and a part of the antigen protein and one selected from a folding factor and its subunit linked thereto via at least one peptide bond.

2. The immunogen as defined in claim 1,
wherein the folding factor is a chaperonin consisting of a plurality of chaperonin subunits.

3. The immunogen as defined in claim 2,
wherein at least two of the chaperonin subunits are serially linked to one another via peptide bonds.

4. The immunogen as defined in claim 2 or 3,
wherein the antigen protein is linked to the N-terminus and/or the C-terminus of the chaperonin subunit.

5. The immunogen as defined in claim 3 or 4,
wherein the antigen protein is linked between the chaperonin subunits.

6. The immunogen as defined in one of claims 2 to 5,
being provided with an amino acid sequence to be cleaved by a protease between the chaperonin subunit and the antigen protein.

7. The immunogen as defined in one of claims 3 to 6,
being provided with an amino acid sequence to be cleaved by a protease between the chaperonin subunits.

8. The immunogen as defined in one of claims 2 to 7,
wherein the chaperonin subunit is derived from one selected from a group consisting of bacteria, archaea and eukaryotes.

9. The immunogen as defined in one of claims 2 to 8,
wherein the antigen protein is accommodated in a chaperonin ring formed by the chaperonin subunits.

10. The immunogen as defined in claim 9,
wherein the chaperonin ring is consisting of 5 to 10 chaperonin subunits.

11. The immunogen as defined in claim 9 or 10,
having two chaperonin rings non-covalently associated on each other's ring plane or each other's side.

12. The immunogen as defined in claim 1,
wherein the folding factor is a foldase.

13. The immunogen as defined in claim 12,
wherein the antigen protein is linked to the N-terminus and/or the C-tenninus of the foldase.

14. The immunogen as defined in claim 12 or 13,
wherein the foldase is a PPIase.

15. The immunogen as defined in claim 14,
wherein the PPIase is derived from one selected from a group consisting of Escherichia coli and archaea.

16. The immunogen as defined in one of claims 1 to 15,
wherein the antigen protein is serotonin receptor 5-HT1aR.

17. The immunogen as defined in claim 16,
wherein the fusion protein comprises either the full-length of serotonin receptor 5-HT1aR or a partial protein consisting of 6 or more amino acid residues thereof.

18. The immunogen as defined in one of claims 1 to 17,
being produced by transcription and translation of a fusion gene comprising a gene encoding one selected from the full-length and a part of the antigen protein and a gene encoding one selected from the folding factor and its subunit.

19. The immunogen as defined in claim 18,
wherein the gene encoding a part of the antigen protein is a gene encoding a partial protein consisting of 6 or more amino acid residues of the antigen protein.

20. A composition for immunological use,
being prepared by mixing of the immunogen as defined in one of claims 1 to 19 with an adjuvant.

21. A method of producing an antibody, the method comprising the steps of:
immunizing an animal except human with the immunogen as defined in one of claims 1 to 19, and
obtaining an antibody specific to the antigen protein from the animal.

22. A method of producing an antibody, the method comprising the steps of:
immunizing an animal except human with the composition as defined in claim 20, and
obtaining an antibody specific to the antigen protein from the animal.
